# EUROPÄISCHE PATENTSCHRIFT

(11) **EP 4 048 209 B1**
(45) Veröffentlichungstag und Bekanntmachung des Hinweises auf die Patenterteilung: **16.04.2025**
(21) Anmeldenummer: 20788760.5
(22) Anmeldetag: 07.10.2020
(51) Int. Cl.: A61F 2/64, A61F 2/70, A61F 5/01, A61F 2/50, A61F 2/66, A61F 2/68, A61F 2/74, A61F 2/76

(54) **ORTHOPÄDIETECHNISCHES GELENK UND VERFAHREN ZU DESSEN STEUERUNG**
ORTHOPAEDIC JOINT AND METHOD FOR CONTROLLING SAME
ARTICULATION ORTHOPÉDIQUE ET SON PROCÉDÉ DE COMMANDE

(30) Priorität: 22.10.2019 DE 102019128508
(43) Veröffentlichungstag der Anmeldung: 31.08.2022
(73) Patentinhaber: Otto Bock Healthcare Products GmbH, 1110 Wien (AT)
(72) Erfinder: SEIFERT, Dirk, 1070 Wien (AT); FUCHS, Florian, 2020 Hollabrunn (AT)
(74) Vertreter: Gramm, Lins & Partner Patent- und Rechtsanwälte PartGmbB
(86) Internationale Anmeldenummer: PCT/EP2020/078102
(87) Internationale Veröffentlichungsnummer: WO 2021/078516

(56) Entgegenhaltungen:
- WO-A1-01/43669
- WO-A1-2011/057790
- WO-A1-2016/169853
- DE-A1- 102009 052 887
- DE-B3- 102016 114 075
- DE-B4- 102009 052 887

## Beschreibung

Die Erfindung betrifft ein orthopädietechnisches Gelenk, insbesondere ein Prothesengelenk oder Orthesengelenk, mit einem Oberteil und einem schwenkbar daran angeordneten Unterteil und einer zwischen dem Oberteil und dem Unterteil angeordneten Widerstandseinrichtung, die einen Widerstand gegen eine Verschwenkbewegung des Oberteils zu dem Unterteil um eine Schwenkachse bereitstellt und eine Verstelleinrichtung aufweist, über die der Widerstand einstellbar ist, wobei die Verstelleinrichtung mit einer Steuerungseinrichtung gekoppelt ist, die mit zumindest einem Sensor gekoppelt ist, sodass über die Steuerungseinrichtung der Widerstand auf Basis von Sensordaten, die von dem zumindest einen Sensor an die Steuerungseinrichtung übermittelt werden, verstellbar ist, wobei in der Steuerungseinrichtung eine Funktion hinterlegt ist, in der das Gelenk in Abhängigkeit von den Sensordaten gegen eine Verschwenkung in zumindest einer Richtung gesperrt ist und die Funktion auf Grundlage der Sensordaten für eine Sperrung aktivierbar und eine Entsperrung deaktivierbar ist. Die Erfindung betrifft ebenfalls ein Verfahren zur Steuerung eines orthopädietechnischen Gelenkes, insbesondere eines Prothesengelenkes oder Orthesengelenkes, mit einem Oberteil und einem schwenkbar daran angeordneten Unterteil und einer zwischen dem Oberteil und dem Unterteil angeordneten Widerstandseinrichtung, die einen Widerstand gegen eine Verschwenkbewegung des Oberteils zu dem Unterteil um eine Schwenkachse bereitstellt und eine Verstelleinrichtung aufweist, über die der Widerstand auf Basis von Sensordaten, die von zumindest einem Sensor an eine mit der Verstelleinrichtung verbundene Steuerungseinrichtung übermittelt werden, dergestalt verstellt wird, dass bei einer Aktivierung einer Funktion das Gelenk gegen eine Verschwenkung gesperrt und bei einer Deaktivierung der Funktion das Gelenk entsperrt wird.

Orthopädietechnische Hilfsmittel wie Prothesen, Orthesen oder auch Exoskelette weisen Gelenke auf, um zwei Komponenten der orthopädietechnischen Hilfsmittel miteinander beweglich zu verbinden. Eine Verschwenkbarkeit der beiden Komponenten zueinander um eine Schwenkachse, die als eine feste Schwenkachse bei einem einachsigen Gelenk oder als eine wandernde Schwenkachse bei einem Mehrlenkersystem ausgebildet sein kann, ermöglicht beispielsweise eine Führung und Unterstützung einer Bewegung durch eine Orthese oder das Ausführen von Bewegungen, die der natürlichen Bewegung entsprechen. Bei der Prothese oder Orthese der unteren Extremität kann damit ein dem natürlichen Bewegungsverhalten angenähertes Bewegungsmuster erreicht werden. Ein Prothesenfuß ist dabei mit einem Unterschenkelschaft oder einem Unterschenkelrohr über ein Knöchelgelenk verbunden. Das Unterschenkelrohr ist an einem Prothesenkniegelenk befestigt, das über einen Oberschenkelschaft an einem Oberschenkelstumpf angeordnet ist. Entsprechendes gilt für Orthesen, die an vorhandenen Gliedmaßen angelegt werden. Das orthopädietechnische Hilfsmittel kann ein oder mehrere Gelenke überbrücken, beispielsweise ein Kniegelenk und/oder ein Knöchelgelenk oder für den Fall einer Anwendung an einer oberen Extremität ein Ellenbogengelenk und/oder ein Handgelenk.

Um die Verschwenkung um die Schwenkachse beeinflussen zu können, sind aus dem Stand der Technik Dämpfer bekannt, die einer Extensionsbewegung und/oder Flexionsbewegung einen Widerstand entgegensetzen. Der Widerstand kann einstellbar sein, ebenfalls ist es möglich, dass der Widerstand sich in Abhängigkeit von Belastungssituationen oder Bewegungsparametern ändert. Bei einer schnellen Bewegung kann z.B. ein größerer Widerstand automatisch bereitgestellt werden, ebenfalls ist es möglich, einen Widerstandsverlauf winkelabhängig oder belastungsabhängig einzustellen oder zu verändern. Die Einstellung kann mechanisch oder auf einen mechatronischen Weg erfolgen. In einer mechatronischen Ausgestaltung des Dämpfers oder der Widerstandseinrichtung werden über Sensoren Bewegungsdaten, Belastungsdaten und/oder auch Positionsdaten aufgenommen und einer Steuerungseinrichtung übermittelt. In dieser wird daraus ein Aktivierungssignal oder Deaktivierungssignal für einen Aktuator oder eine Verstelleinrichtung generiert, um den jeweiligen Widerstand zu verringern oder zu vergrößern. Dies kann beispielsweise durch eine Veränderung eines Strömungsquerschnittes bei einem hydraulischen Dämpfer oder eine Veränderung eines Magnetfeldes bei magnetorheologischen Widerstandseinrichtungen erfolgen. Auch kann die Widerstandseinrichtung als Elektromotor ausgestaltet sein, der im Generatorbetrieb einen Widerstand gegen eine Verschwenkbewegung bereitstellt.

Insbesondere bei computergesteuerten Gelenken oder Gelenkeinrichtungen mit Sensoren können unterschiedliche Steuerungsprogramme hinterlegt werden, um ein angepasstes Widerstandsverhalten für unterschiedliche Belastungen oder Bewegungssituationen bereitstellen zu können. Für das Gehen in der Ebene kann für einen Schrittzyklus ein Widerstandsprofil erzeugt werden, das sich von dem Gehen auf einer Rampe unterscheidet. Darüber hinaus sind in computergesteuerten Gelenken Funktionen für Sondersituationen hinterlegt, bei denen beispielsweise durch wiederholtes Ausführen eines bestimmten Bewegungsmuster ein Gelenk gesperrt wird oder nach einem bestimmten Zeitablauf eine Sperrung aktiviert oder deaktiviert wird. Das "C-Leg^{®}" der Firma Ottobock verfügt über eine Stehfunktion, die über ein Bewegungsmuster aktiviert werden kann. Darüber hinaus gibt es die Wahl einer intuitiven Stehfunktion, die das Gelenk blockiert, wenn es in gebeugter Position ruhig gehalten wird. Ändert sich die Lage des Beines, wird die Stehfunktion deaktiviert.

Die Parameter für das Aktivieren und Deaktivieren von Sonderfunktionen sind entweder fest vorgegeben oder werden für den Patienten individuell ermittelt und einmalig eingestellt.

Die DE 10 2009 052 887 A1 betrifft ein Verfahren zur Steuerung eines orthetischen oder prothetischen Gelenkes einer unteren Extremität mit einer Widerstandseinrichtung, der zumindest ein Aktuator zugeordnet ist, über den der Beuge- und/oder Streckwiderstand in Abhängigkeit von Sensordaten verändert wird.

Die WO 01/43669 A1 betrifft eine Prothese oder Orthese mit zwei gelenkig aneinander festgelegten Teilen und einer Steuerungseinrichtung für das Gelenk mit zumindest einem Sensor, der die Stellung zumindest eines Teiles in Relation zu einer festliegenden Linie, insbesondere der Gravitationsrichtung, ermittelt. Der Sensor ist mit der Steuerungseinrichtung gekoppelt und auf Grundlage der ermittelten Daten wird das Gelenk beeinflusst, beispielsweise ein Widerstand verändert.

Die WO 2011/057790 A1betrifft ein Verfahren und eine Vorrichtung zur Steuerung eines künstlichen orthetischen oder prothetischen Gelenkes einer unteren Extremität mit einer Widerstandseinrichtung, der zumindest ein Aktuator zugeordnet ist, über den der Beuge- und/oder Streckwiderstand in Abhängigkeit von Sensordaten verändert wird, wobei während der Benutzung des Gelenkes über Sensoren Zustandsinformationen bereitgestellt werden. Der Widerstand in der Standphase oder während des Stehens wird von einem Ausgangswert in Abhängigkeit von der Bodenreaktionskraft bis zu einer Sperre des Gelenkes erhöht. Zudem ist eine Einstellvorrichtung vorgesehen, über die belastungsabhängige Widerstandsänderung aktivierbar und/oder deaktivierbar ist.

Aufgabe der vorliegenden Erfindung ist es, ein Gelenk und ein Verfahren zum Steuern eines Gelenkes bereitzustellen, das leichter an die Bedürfnisse des jeweiligen Nutzers anpassbar ist.

Diese Aufgabe wird durch ein Gelenk mit den Merkmalen des Hauptanspruches und ein Verfahren mit den Merkmalen des nebengeordneten Anspruchs gelöst. Vorteilhafte Ausgestaltungen und Weiterbildungen der Erfindung sind in den Unteransprüchen, der Beschreibung und den Figuren offenbart.

Das orthopädietechnische Gelenk mit einem Oberteil und einem schwenkbar daran angeordneten Unterteil und einer zwischen dem Oberteil und dem Unterteil angeordneten Widerstandseinrichtung, die einen Widerstand gegen eine Verschwenkbewegung des Oberteils zu dem Unterteil um eine Schwenkachse bereitstellt und eine Verstelleinrichtung aufweist, über die der Widerstand einstellbar ist, wobei die Verstelleinrichtung mit einer Steuerungseinrichtung gekoppelt ist, die mit zumindest einem Sensor gekoppelt ist, sodass über die Steuerungseinrichtung der Widerstand auf Basis von Sensordaten, die von dem zumindest einen Sensor an die Steuerungseinrichtung übermittelt werden, verstellbar ist, wobei in der Steuerungseinrichtung eine Funktion hinterlegt ist, in der das Gelenk in Abhängigkeit von den Sensordaten gegen eine Verschwenkung in zumindest einer Richtung gesperrt ist, wobei die Funktion auf Grundlage der Sensordaten für eine Sperrung aktivierbar und eine Entsperrung deaktivierbar ist, sieht vor, dass zumindest ein Parameter für die Aktivierung und zumindest ein Parameter, der von dem Parameter für die Aktivierung verschieden ist, für die Deaktivierung der Funktion miteinander korreliert sind. Mit einem solchen Gelenk ist es möglich, die Charakteristiken für das Aktivieren und Deaktivieren der Sperre einzustellen. Das Verhalten des Patienten zum Auslösen einer Sperrung oder zum Aufheben einer Sperrung kann über mehrere Parameter korreliert werden. Die Parameter oder der Parameter, der zum Sperren des Gelenkes führt, kann beispielsweise hinsichtlich der Empfindlichkeit verändert werden und besonders schnell zu einem Sperren des Gelenkes führen; gleichzeitig wird derjenige Parameter, der zum Deaktivieren des Gelenkes führt, mitverändert. Der jeweilige Parameter kann dabei aus mehreren Sensorwerten oder Eigenschaften zusammengesetzt sein, beispielsweise aus einem Zeitfaktor und einem Bewegungsfaktor. Wird beispielsweise für eine bestimmte Zeit das Gelenk nicht bewegt, kann dies als Parameter dafür dienen, dass ein Gelenk gesperrt werden soll. Eine wenig empfindliche Parametrisierung sieht vor, dass erst nach einem vollständigen Stillhalten über einen vergleichsweise langen Zeitraum eine Verriegelung stattfindet. Eine empfindlichere Parametrisierung kann vorsehen, dass in einem gleichen Zeitraum geringe Relativbewegungen oder Verschwenkung erlaubt sind oder aber das beim vollständigen Stillstand des Gelenkes und kurzer Dauer dieses Stillstandes oder aber bei einer kurzen, nahezu vollständigen Ruhigstellung des Gelenkes bereits die Sperrung aktiviert oder veranlasst wird. Umgekehrt kann die entsprechende Deaktivierung ebenfalls empfindlich eingestellt werden, beispielsweise durch das Absenken eines Schwellwertes für die Raumlageänderung oder eine geringe Veränderung einer axialen Belastung.

Ein Gelenk kann dadurch gesperrt werden, dass es gegen eine Relativbewegung von Oberteil und Unterteil formschlüssig verriegelt wird. Ebenso ist es möglich, dass die Widerstandseinrichtung, beispielsweise ein Hydraulikdämpfer, eine Bremse, ein magnetorheologischer Dämpfer, eine Linearhydraulik und/oder ein Elektromotor in Generatorbetrieb auf einen so hohen Widerstandswert eingestellt wird, dass bei einer normalen Belastung nicht mit einer Verlagerung zu rechnen ist oder aber eine Relativbewegung von Oberteil und Unterteil zueinander nur sehr langsam oder unter Aufbringung sehr hoher Kräfte und Momente möglich ist. Eine Sperrung kann somit eine vollständige Immobilisierung des Gelenkes umfassen oder einen sehr hohen Widerstand gegen eine Flexion und/oder Extension bedeuten.

Als Parameter für die Aktivierung der Funktion kann die Zeitdauer eines statischen Zustandes der Gelenkeinrichtung hinterlegt sein. Ab einem bestimmten Schwellwert des statischen Zustandes geht die Steuerungseinrichtung davon aus, dass das Gelenk gesperrt werden soll und veranlasst durch die Verstelleinrichtung die Erhöhung des Widerstandes gegen eine Flexion und/oder Extension durch die Widerstandseinrichtung. Dazu können Ventile geschlossen, Magnetfelder angelegt oder abgeschaltet oder auch mechanische Widerstände wie Bremsen oder Formschlusselemente aktiviert oder in Eingriff gebracht werden. Neben der Zeit und des Vorhandenseins eines statischen Zustandes können auch weitere Sensorwerte oder Zustände in den Parameter einfließen, beispielsweise Belastungen, Änderungen von Belastungen, Winkelstellungen von Gelenkkomponenten zueinander oder auch Absolutstellungen von Gelenkkomponenten im Raum.

Als Parameter für die Aktivierung oder Deaktivierung der Funktion können eine Bewegung des Unterteils, eine Bewegung des Oberteils oder eine Belastung des Gelenkes oder einer Gelenkkomponente oder Änderungsraten der Bewegung oder der Belastung hinterlegt sein. Darüber hinaus können noch weitere Parameter oder Kenngrößen hinterlegt sein, über die es möglich ist, eine Aktivierung oder Deaktivierung der Sperrung durch die Widerstandseinrichtung zu bewirken. Beispielweise kann über einen Sensor eine sehr hohe Beschleunigung in einer Richtung, die bei einem normalen Bewegungsablauf nicht auftreten würde, erfasst werden. Wird beispielsweise ein Gelenk mehrfach in entgegengesetzten Richtungen hin und her bewegt, können diese Beschleunigungen und Bewegungsrichtungsänderungen als Grundlage für eine Verriegelung oder Sperrung oder Erhöhung des Widerstandes auf einen vorgesehenen Wert verwendet werden.

Als Parameter für die Deaktivierung der Funktion kann beispielsweise eine Raumlage des Oberteils, eine Raumlage des Unterteils und/oder eine Raumlage eine Verbindungslinie zwischen zwei Punkten proximal und distal der Schwenkachse hinterlegt sein. Die Verbindungslinie zwischen zwei festgelegten Punkten kann als sogenannte Gelenksehne vorab definiert sein. Die Raumlage der Gelenksehne lässt Rückschlüsse auf die Bewegungssituation zu. Beispielsweise kann über die Raumlage einer Beinsehne zwischen einem Oberschenkel und einem Unterschenkel, beispielsweise zwischen dem Hüftgelenk und dem Knöchelgelenk, ermittelt werden, ob ein Nutzer oder Patient steht, liegt oder sitzt. Ist ein Kniegelenk in einer gestreckten, stehenden Position gesperrt, kann durch eine Vorwärtsneigung oder Rückwärtsneigung des Oberteils zusammen mit dem Unterteil, beispielsweise beim Hinsetzen, eine Entsperrung der Gelenkeinrichtung durch Lösen der Widerstandseinrichtung oder Verringerung des Flexionswiderstandes herbeigeführt werden

Eine Weiterbildung der Erfindung sieht vor, dass Schwellwerte für die Parameter für die Aktivierung und/oder Deaktivierung hinterlegt sind. Die Schwellwerte können in einer Speichereinrichtung, die mit der Steuerungseinrichtung gekoppelt oder darin integriert ist, abgelegt sein. Die Schwellwerte können veränderlich sein, sodass für den Nutzer oder einen Orthopädietechniker die Möglichkeit gegeben ist, eine individuelle Anpassung an den jeweiligen Nutzer vorzunehmen. Die Schwellwerte können stufenlos oder in Stufen verstellt werden. Eine stufenlose Verstellung kann beispielsweise bei einem zeitlichen Parameter oder einer zeitlichen Kenngröße sinnvoll sein. Eine stufenweise Verstellung erleichtert die Spürbarkeit unterschiedlicher Schwellwerte und vereinfacht die Einstellung. Insbesondere bei mehreren Messwerten, die zu einem Parameter zusammengefasst werden, kann durch eine Zusammenfassung der Werte und eine vorgegebene Abstufung das Einstellen erleichtert werden. Beispielsweise kann eine Empfindlichkeit der Verriegelung in mehreren Stufen von stabil über normal bis empfindlich durch eine Auswahl von Symbolen oder Icons erleichtert werden. Eine stufenlose Verstellung kann über berührungsempfindliche Einstellelemente an einer Ausgabeeinrichtung oder über Regler die Drehknöpfe oder Schieberegler, die auch digitalisiert und virtuell dargestellt werden können, erfolgen.

Die Schwellwerte für die Aktivierung und Deaktivierung können positiv korreliert sein. Bei einer empfindlichen Einstellung für die Aktivierung einer Sperre bedarf es entsprechend einer geringfügigen Aktion oder eines niedrigschwelligen Impulses, um die Deaktivierung vorzunehmen. Wird beispielsweise ein Gelenk nach kurzer Zeit mit einer teilweisen Ruhigstellung verriegelt, bedeutet eine positive Korrelation mit der Deaktivierung, dass eine geringfügige Verlagerung oder eine geringfügige Entlastung oder Belastung oder Beschleunigung ausreicht, um die Sperrung wieder aufzuheben und eine Flexion und/oder Extension zuzulassen.

Die Steuerungseinrichtung kann mit einer Anzeige und einem Bedienelemente zur Veränderung der Parameter und/oder der Schwellwerte der Parameter verbunden sein, sodass es für einen Anwender oder einen Orthopädietechniker einfach ist, eine Anpassung an den jeweiligen Nutzer vorzunehmen. Ein übergeordnetes Kriterium wie "Empfindlichkeit" kann als sogenannter Masterregler eine einfache Veränderung ermöglichen. Darüber hinaus ist es möglich, dass die einzelnen Werte oder Einflussgrößen für die Parameter wie Zeit, Belastung, Verlagerung, Größe der Verlagerung, Geschwindigkeiten, Beschleunigungen, Momente oder Stellungen im Raum individuell angepasst und einzeln miteinander kombiniert werden können.

Zur Bereitstellung der notwendigen Sensorwerte können ein Gyroskop, eine Inertial-Messeinheiten, ein Winkelsensor, ein Kraftsensor und/oder ein Zeitsensor mit der Steuerungseinrichtung gekoppelt sein, um die notwendigen Sensordaten der Steuerungseinrichtung zur Verfügung stellen zu können. Es können auch mehrere dieser Sensoren dem orthopädietechnischen Gelenk oder dem orthopädietechnischen Hilfsmittel zugeordnet sein, insbesondere mit dem Gelenk oder dem orthopädietechnischen Hilfsmittel verbunden sein. Es besteht auch die Möglichkeit neben einer direkten Befestigung der Sensoren oder des Sensors an dem Gelenk oder an weiteren Komponenten, die an dem Gelenk befestigt sind, die Sensoren von dem orthopädietechnischen Hilfsmittel getrennt an dem Nutzer anzubringen und die Sensordaten per Kabel oder kabellos an die Steuerungseinrichtung zu übermitteln. In der Steuerungseinrichtung sind die notwendigen Komponenten zur elektronischen Verarbeitung der Sensordaten untergebracht, insbesondere zumindest ein Prozessor, zumindest ein Speicher, eine Energieversorgung, Speicherelemente für die benötigte Software sowie eine Einrichtung zur Aufnahme von Sensordaten sowie Ausgabe von Steuerungsdaten, mit denen die Verstelleinrichtung für die Widerstandseinrichtung aktiviert oder deaktiviert werden kann.

Das Verfahren zur Steuerung eines orthopädietechnischen Gelenkes mit einem Oberteil und einem schwenkbar daran angeordneten Unterteil und einer zwischen dem Oberteil und dem Unterteil angeordneten Widerstandseinrichtung, die einen Widerstand gegen eine Verschwenkbewegung des Oberteils relativ zu dem Unterteil um eine Schwenkachse bereitstellt und eine Verstelleinrichtung aufweist, über die der Widerstand auf Basis von Sensordaten, die von zumindest einem Sensor an eine mit der Verstelleinrichtung verbundenen Steuerungseinrichtung übermittelt werden, dergestalt verstellt wird, dass bei Aktivierung einer Funktion das Gelenk gegen eine Verschwenkung gesperrt und bei Deaktivierung der Funktion entsperrt, sieht vor, dass zumindest ein Schwellwert für einen Parameter für die Aktivierung und zumindest ein Schwellwert für einen Parameter für die Deaktivierung der Funktion miteinander korreliert werden. Die Schwellwerte für die Deaktivierung und die Aktivierung sind in der Steuerungseinrichtung abgelegt und werden mit den Sensordaten verglichen. Die Sensordaten können entweder von den Sensoren bereits aufbereitet übermittelt oder als sogenannte Rohdaten in der Steuerungseinrichtung aufbereitet und in aufbereiteter Form mit den in der Software abgelegten Schwellwerten verglichen werden. Wird ein Parameter oder ein Schwellwert für einen Parameter für die Aktivierung verändert, führt dies zu einer Veränderung des Parameters für die Deaktivierung und gegebenenfalls umgekehrt. In der Software in der Steuerungseinrichtung wird für den Orthopädietechniker und/oder den Nutzer ein Schwellwert implementiert, der eine Einstellbarkeit oder eine Auswahl über die Stabilität bzw. über das Ansprechverhalten der Steuerung und damit das Ansprechverhalten der Verriegelung ermöglicht. Der Einstellwert kann dabei mehrere Parameter der Funktion, beispielsweise einer Stehfunktion, gleichzeitig und gut aufeinander abgestimmt verändern. Wird beispielsweise ein feines oder empfindliches Ansprechverhalten gewünscht, um die Funktion zu aktivieren, wird die Funktion so parametrisiert, dass beispielsweise eine kurze Ruheposition ausreicht, um eine Sperre gegen eine Flexion und/oder Extension zu erreichen. Gleichzeitig werden die notwendigen Schwellwerte für die Aufhebung der Sperre, also für die Deaktivierung der Sperre und damit eine Freischaltung oder eine Verringerung des Sperrwiderstandes gesenkt, wenn eine positive Korrelation vorhanden ist. Die Schwellwerte sind beispielsweise ein Bewegungsumfang oder eine Winkelgeschwindigkeit eines Gelenksegmentes. Bei einer negativen Korrelation werden die Schwellwerte für die Aufhebung der Sperre erhöht. Umgekehrt kann bei einer gewünschten hohen Stabilität und einer gewünschten Verriegelung nur in eindeutigen Fällen die Funktion so parametrisiert werden, dass eine längere Ruheposition oder eine längere Zeitdauer eines statischen Zustandes notwendig ist, um eine Sperre zu erreichen. Bei einer positiven Korrelation der Deaktivierungsparameter ist entsprechend ein größeres Bewegungsausmaß, eine größere Winkelgeschwindigkeit oder eine höhere Beschleunigung notwendig, um die Sperre aufzuheben oder den Widerstand auf ein entsprechendes Niveau abzusenken.

Wenn die Schwellwerte oder die Parameter für die Aktivierung und Deaktivierung positiv miteinander korreliert werden, ist dies für einen Anwender in der Regel vorteilhaft, da beispielsweise unsichere Patienten eindeutige Situationen zum Verriegeln und Entriegeln bevorzugen, während Nutzer von Gelenken, die eine sensible Verriegelung bevorzugen, in der Regel auch eine empfindliche Steuerung für eine Entriegelung bevorzugen.

Die Funktion kann insbesondere auf der Grundlage der Zeitdauer eines statischen Zustandes der Gelenkeinrichtung aktiviert werden, insbesondere auf der Grundlage der Zeitdauer eines statischen Zustandes in einer bestimmten Orientierung einer Komponente oder in einer bestimmten Orientierung einer Gelenkssehne.

Die Funktion kann auf der Grundlage einer Bewegung, also einer Aufhebung des statischen Zustandes, des Unterteils, des Oberteils oder über eine Belastung des Gelenkes oder einer Gelenkkomponente deaktiviert werden, wobei anderseits eine Belastung des Gelenkes auch ein Kriterium für eine Aktivierung einer Sperre sein kann. Die Aufhebung der Sperre kann auch über die Erfassung von Geschwindigkeiten oder Beschleunigungen oder über besondere Orientierungen von Gelenkkomponenten im Raum oder zueinander erfolgen.

Insbesondere kann eine Funktion, die eine Sperrung bewirkt, auf der Grundlage einer Raumlage des Oberteils, des Unterteils und/oder einer Verbindungslinie zwischen zwei Punkten proximal und distal der Schwenkachse deaktiviert werden.

Die Schwellwerte für die Parameter für die Aktivierung und oder Deaktivierung sind bevorzugt veränderlich, insbesondere stufenweise oder stufenlos verstellbar. Die Parameter können beispielsweise auf einem berührungsempfindlichen Display angezeigt werden, sodass durch Verstellen eines dargestellten Schiebereglers oder Drehreglers oder bei einer stufenweisen Erstellung durch Berühren eines Icons eine entsprechende Parameterverstellung oder Schwellwertverstellung des jeweiligen Parameters erfolgt. Über ein oder mehrere Icons auf dem Display kann der Nutzer oder Orthopädietechniker über die Schwellwerte und deren Eigenschaften aufgeklärt werden, sodass die jeweilige Person die notwendigen Informationen darüber erhält, welche Einstellungen was für Auswirkungen haben.

Nachfolgend werden Ausführungsbeispiele der Erfindung anhand der beigefügten Figuren näher erläutert. Es zeigen:
Figur 1 - ein orthopädietechnisches Hilfsmittel in Gestalt eine Orthese in einer ersten Position;
Figur 2 - ein orthopädietechnisches Hilfsmittel in Gestalt einer Prothese in einer ersten Stellung;
Figuren 3 bis 6 - eine Prothese gemäß Figur 2 in unterschiedlichen Stellungen;
Figur 7 - eine Orthese gemäß Figur 1 während der Benutzung;
Figur 8 - eine schematische Darstellung der Verstellung einer Sperrung und Entsperrung;
Figur 9 - eine schematische Darstellung eines Prothesenkniegelenkes; sowie
Figur 10 - verschiedene Ausführungsformen von Widerstands- und Verstelleinrichtungen.

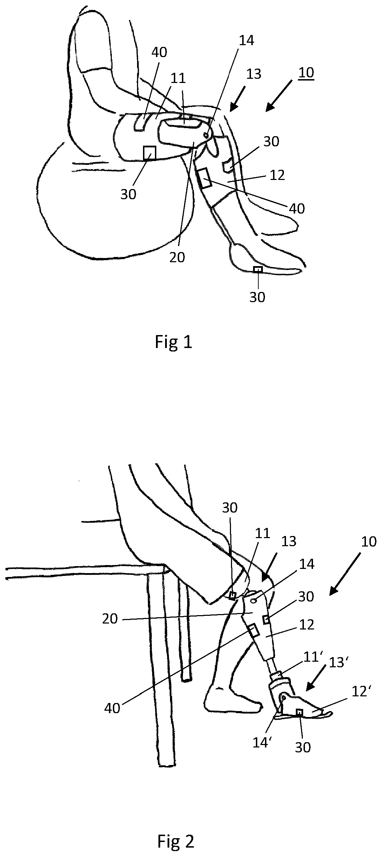

Figur 1 zeigt in einer schematischen Darstellung ein orthopädietechnisches Hilfsmittel in Gestalt einer Orthese 10 mit einem Oberteil 11, das über ein Gelenk 13 mit einem Unterteil 12 verbunden ist. Das Unterteil 12 ist um eine Schwenkachse 14 mit dem Oberteil 11 verbunden. Das Oberteil 11 der Beinorthese ist als Schale oder Schiene zur Aufnahme eines Oberschenkels ausgebildet, das Unterteil 12 ist zur Anlage an einem Unterschenkel ausgebildet und weist ein Fußteil auf, das an dem Unterteil 12 angeformt oder alternativ daran befestigt ist. An dem Oberteil 11 ist eine Widerstandseinrichtung 20 angeordnet, über die ein Verschwenkungswiderstand des Unterteils 12 relativ zu Oberteil 11 verstellt werden kann. Die Widerstandseinrichtung 20 kann insbesondere zur Sperrung einer Verschwenkbewegung eingesetzt werden und ist beispielsweise als ein Hydraulikdämpfer, eine Bremse, eine magnetorheologische Widerstandseinrichtung oder dergleichen ausgebildet. Sowohl an dem Oberteil 11 als auch an dem Unterteil 12 sind Sensoren 30 angeordnet, die Informationen über die gegenwärtige Raumlage, die Position relativ zueinander und gegebenenfalls Belastungen, Winkelstellungen und/oder Beschleunigungen der jeweiligen Komponenten aufnehmen können. Die Sensoren 30 sind mit einer Steuerungseinrichtung 40 gekoppelt, entweder per Kabel oder drahtlos. Innerhalb der Steuerungseinrichtung 40 werden die Sensordaten ausgewertet und mit Schwellwerten verglichen, die in Programmen oder Speichern innerhalb der Steuerungseinrichtung 40 abgelegt sind. Werden die entsprechenden Kriterien für eine Sperrung oder Entsperrung des Gelenkes 13 erfüllt, gibt die Steuerungseinrichtung 40 einen Steuerbefehl an eine Verstelleinrichtung 60 aus, die an der Widerstandseinrichtung 20 angeordnet ist. Die Verstelleinrichtung, beispielsweise ein Motor oder Aktuator zur Verstellung eines Ventils, eine Spule zur Veränderung eines Magnetfeldes oder eine andere Betätigungseinrichtung oder ein anderes Beeinflussungselement, wirkt dann innerhalb der Widerstandseinrichtung 20 oder auf die Widerstandseinrichtung 20 und sperrt oder entsperrt das Gelenk 13.

In der Figur 2 ist ein korrespondierendes orthopädietechnisches Hilfsmittel in Gestalt einer Prothese dargestellt. Statt einer Oberschenkelschale ist das Oberteil 11 als ein Oberschenkelschaft zur Aufnahme eines Oberschenkelstumpfes ausgebildet, das Gelenk 13 ist als Prothesenkniegelenk ausgebildet, das Unterteil 12 als ein Unterschenkelrohr oder Unterschenkelteil. An dem Unterschenkelteil 12 ist an dem distalen Ende ein Prothesenfuß schwenkbar an einem Gelenk 13 in Gestalt eines Prothesenknöchelgelenkes angeordnet. Die Verschwenkung erfolgt um eine Schwenkachse 14', sodass das Unterschenkelteil 12 hinsichtlich des Prothesenkniegelenkes 13 als Unterteil und hinsichtlich des Prothesenknöchelgelenkes 13 als Oberteil 11' anzusehen ist. Das Fußteil ist dann das Unterteil 12'. Auch an der Prothese sind Sensoren 30 angeordnet, an dem Oberschenkelschaft als Oberteil 11 beispielsweise zumindest ein Gyroskop, ein Beschleunigungssensor und/oder Winkelsensor, an dem Unterschenkelteil als Unterteil 12 beispielsweise ein Raumlagesensor oder eine IMU, ein Axialkraftsensor oder dergleichen, an dem Fußteil kann beispielsweise ein Momentensensor und/oder Kraftsensor angeordnet sein. Es können mehrere Sensoren an der jeweiligen Komponente angeordnet oder dieser zugeordnet sein. Darüber hinaus ist in der Steuerungseinrichtung 40 ein Zeitsensor oder ein Zeitglied angeordnet oder ausgebildet, über das eine Zustandsveränderung oder die Dauer eines erfassten Zustandes ermittelt wird. In den Ausführungsformen der Figuren 1 und 2 befindet sich der Nutzer des orthopädietechnischen Hilfsmittels 10 in einer sitzenden Position, in der das Kniegelenk eingebeugt ist. Die eingebeugte Stellung wird beispielsweise über einen Winkelsensor ermittelt, der die Winkelstellung von dem Oberteil 11 und Unterteil 12 im Raum oder zueinander detektiert. Darüber hinaus ist das Fußteil oder der Prothesenfuß entlastet, da ein Teil des Körpergewichtes aufgrund der sitzenden Position nicht in das orthopädietechnische Hilfsmittel 10 eingeleitet wird. Diese geringe Axialbelastung wird beispielsweise durch einen Kraftsensor in dem Fußteil oder dem Prothesenfuß erfasst. Für den Nutzer kann es angenehm sein, wenn ein Gelenk 13, 13', also das Orthesen- oder Prothesenkniegelenk beziehungsweise das Prothesenknöchelgelenk gesperrt wird, wenn sich der Nutzer über einen gewissen Zeitraum in einer solchen Position befindet. Anhand vorgegebener Parameter wie beispielsweise Axialbelastung, Winkelstellung und/oder Dauer eines statischen Zustandes kann eine Funktion aktiviert werden, mit der das jeweilige Gelenk 13, 13' gesperrt wird. Zur Entsperrung des Gelenkes 13, 13' ist es entsprechend notwendig, eine Situationsänderung zu detektieren. Die Sensorwerte und Parameter für das Deaktivieren der Sperre, also für das Entsperren des jeweiligen Gelenkes 13, 13', werden ebenfalls festgelegt, beispielsweise ein Anstieg der Axialbelastung in dem Fußteil oder Prothesenfuß, eine Raumlageänderung des Oberteils 11 oder ein Moment um eine Schwenkachse 14, 14'. Werden hier die vorgegebenen Schwellwerte erreicht, gibt die Steuerungseinrichtung 40 ein entsprechendes Signal aus und die Widerstandseinrichtung 20 wird durch die Verstelleinrichtung 60 entsperrt.

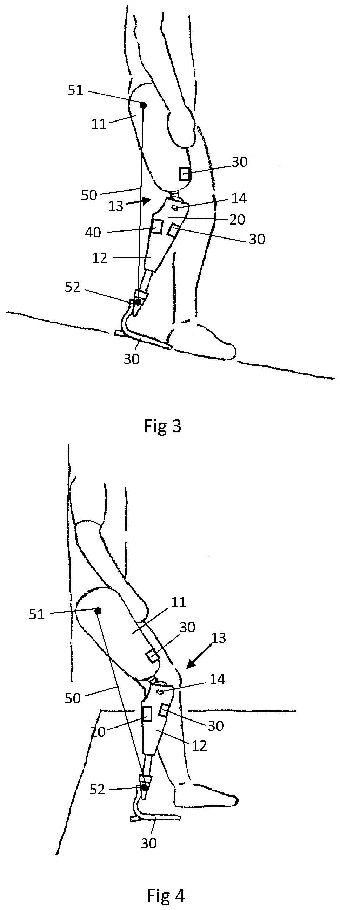

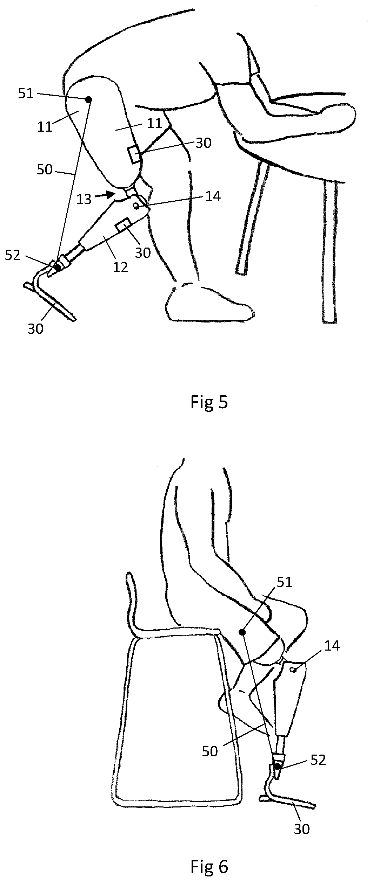

In den Figuren 3 bis 6 sind unterschiedliche Nutzungssituationen des orthopädietechnischen Hilfsmittels 10 am Beispiel eines Prothesenbeines mit einem Oberschenkelschaft, einem Prothesenkniegelenk, einem Unterschenkelteil sowie einem gelenklosen Prothesenfuß gezeigt. In der Figur 3 steht ein Nutzer auf einer geneigten Ebene, in der Figur 4 lehnt der Nutzer an einer Wand, in der Figur 5 lehnt der Nutzer sich nach vorne und stützt sich auf einem Tisch ab, in der Figur 6 sitzt der Nutzer auf einem erhöhten Stuhl. Der grundsätzliche Aufbau der Prothese gemäß der Figuren 3 bis 6 entspricht dem der Prothese gemäß Figur 2, der Unterschied liegt in der Ausgestaltung des Orthesenfußes als gelenkloser Fuß, also ohne zweites Gelenk 13' und eine Schwenkachse 14'. In den Figuren sind nicht sämtliche Komponenten eingezeichnet, um die Übersichtlichkeit zu verbessern.

In den Figuren 3 bis 6 ist eine Verbindungslinie 50 zwischen zwei Punkten 51, 52 an der Prothese eingezeichnet. Der proximale Punkt 51 ist im Bereich des Trochanter Major positioniert, der distale Punkt 52 ist im Bereich des natürlichen Knöchelgelenkes an dem distalen Ende des Unterschenkelteils 12 positioniert. Die Verbindungslinie 50 stellt die sogenannte Beinsehne dar, deren Orientierung und Länge Rückschlüsse auf die absolute Position des Beines, der jeweiligen Komponenten sowie die Bewegungssituation zulässt. Die Raumlage der Verbindungslinie 50 oder Beinsehne kann anhand der Raumlage des Oberteils 11 und/oder des Unterteils 12 sowie der bekannten Entfernungen der Punkte 51, 52 zu der Schwenkachse 14 sowie der Winkelstellung zwischen Oberteil 11 und Unterteil 12 berechnet werden. In der Figur 3 ist die Beinsehne 50 leicht in Gehrichtung nach vorne gekippt und weist aufgrund der geringen Einbeugung in dem Kniegelenk 13 eine nahezu maximale Länge auf. Die Einbeugung in dem Kniegelenk in der Figur 4 ist größer als in der Figur 3, jedoch ist die Beinsehne 50 nach hinten geneigt. Die Beinsehne 50 in der Position gemäß Figur 5 ist vergleichsweise stark nach vorne geneigt und verkürzt gegenüber der Position gemäß Figur 3, die Raumneigung der sitzenden Position gemäß Figur 6 entspricht ungefähr der Neigung der Beinsehne 50 gemäß Figur 4, jedoch ist die Beinsehne 50 aufgrund der größeren Kniebeugung wesentlich kürzer. Darüber hinaus sind in allen Positionen unterschiedliche Axialkräfte beispielsweise über einen Kraftsensor 30 im Fußteil erfassbar, ebenso sind die Momente um die Kniegelenksachse 14 in jeder Nutzungssituation unterschiedlich.

Um beispielsweise den Zeitpunkt der Sperrung des Prothesenkniegelenkes 13 in der jeweiligen Situation an die Wünsche oder Bedürfnisse des jeweiligen Nutzers anpassen zu können, ist der Steuerungseinrichtung 40 ein Bedienelement zugeordnet, das unmittelbar an der Prothese angeordnet sein kann. Alternativ kann das Bedienelement in einer drahtlosen Kommunikation zu der Steuerungseinrichtung 40 stehen und beispielsweise eine Anwendung auf einem Mobiltelefon, einer Fernbedienung, einem Computer oder dergleichen sein. Das Bedienelement kann auch als Drehregler, Schieberegler oder andere Einstelleinrichtung ausgebildet sein.

Befindet sich der Nutzer beispielsweise in einer Position gemäß Figur 4, kann dies bedeuten, dass der Nutzer sich ausruhen möchte. Dazu ist es hilfreich, wenn das Gelenk 13 gesperrt wird. Es kann der Wunsch bestehen, dass diese Sperrung durch die Widerstandseinrichtung 20 sehr schnell ausgeführt wird, um die Anstrengungen des Nutzers zu reduzieren. Verändert der Nutzer seine Position, soll die Prothese ihre normalen Eigenschaften und ihre Beweglichkeit beispielsweise für das Gehen in der Ebene möglichst schnell wieder ausführen. Dementsprechend wird der Zeitraum für die Sperrung nach Erkennen der Position gemäß Figur 4, beispielsweise durch Erkennen der Rückwärtsneigung der Beinsehne 50 bei einem Drehmoment um die Schwenkachse 14 und einer ansonsten statischen Position der Prothese, kurz eingestellt. Zum Entsperren der Widerstandseinrichtung 20 bedarf es dann einer nur geringfügigen Veränderung der Raumlage der Beinsehne 50, beispielsweise ein geringes Verkippen nach vorne.

Befindet sich der Nutzer in einer vorgebeugten Stellung gemäß Figur 5, beispielsweise beim Arbeiten an einem niedrigen Tisch, kann eine schnelle Verriegelung des Kniegelenkes 13 gewünscht, jedoch eine schnelle Entriegelung und damit eine Gefahr einer Instabilität unerwünscht sein. Für eine solche Position der Beinsehne 50 mit einer entsprechenden Belastungssituation kann eine negative Rückkopplung vorteilhaft sein, also eine schnelle Verriegelung bei einer langsamen oder trägen Entriegelung.

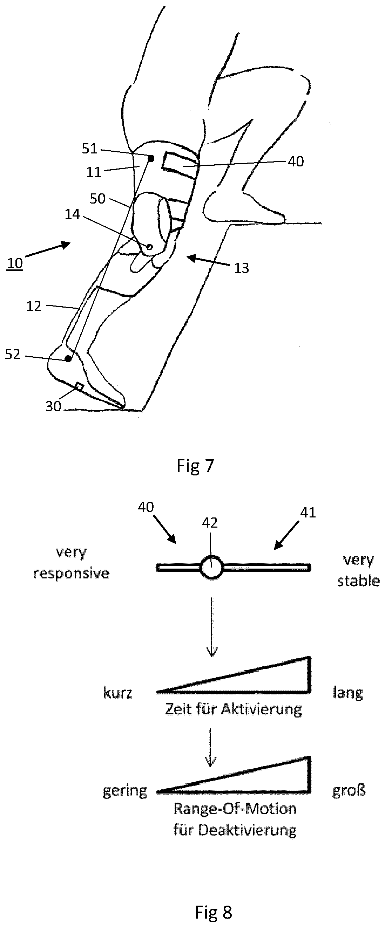

**In** der Figur 7 ist das orthopädietechnische Hilfsmittel 10 als Orthese dargestellt, auch in dieser Stellung kann eine schnelle Verriegelung des Orthesenkniegelenkes 13 bei einer statischen Position, beispielsweise wenn der Nutzer in dieser Position arbeitet, sinnvoll sein. Neben der Orientierung der Beinsehne 50 kann deren statischer Zustand über einen gewissen Zeitraum als Indikator dienen, dass eine Sperrung stattfinden soll. Darüber hinaus können Axialbelastungen oder Momente über Sensoren 30 im Fußteil erfasst werden, sodass bei einer hohen Belastung auf dem Bein mit der Orthese eine schnelle Verriegelung gewünscht sein kann. Ändert sich die Belastungssituation und die Nutzungssituation, kann eine schnelle Entriegelung gewünscht sein. Hierzu kann an der Steuerungseinrichtung 40, die sich an dem Oberschenkelteil oder Oberteil 11 der Orthese befindet, ein Bedienfeld, ein Display oder ein Bedienelement angeordnet sein, so dass einfach eine Verstellung der Empfindlichkeit durch den Nutzer während der Benutzung stattfinden kann.

Figur 8 zeigt exemplarisch einen Teil einer Steuerungseinrichtung 40 mit einem Bedienelement 42, das als berührungsempfindliches Element in einer Anzeigeeinrichtung 41, beispielsweise einem Display, ausgebildet ist. Das Bedienelement 42 kann auch als mechanisches Bedienelement, beispielsweise als Regler, Schieber oder Schalter ausgebildet sein. Die Stellung des Bedienelementes 42 kann zwischen den beiden Extremwerten für sehr empfindlich (very responsive) mit einer schnellen Verriegelung und einer entsprechend schnellen Entriegelung und sehr stabil (very stable) verschoben werden. Die Verstellung kann stufenlos oder stufenweise erfolgen. Unterhalb des Bedienelementes 42 wird angezeigt, wie sich dadurch die Zeit für die Aktivierung der Sperre durch die Widerstandseinrichtung 20 verändert, nämlich von kurz zu lang. Für die Deaktivierung der Sperre ist darunter der in diesem Ausführungsbeispiel dazugehörige Parameter dargestellt, nämlich der Grad der Bewegung, beispielsweise die Verschwenkung einer Komponente oder die Verschwenkung der Beinsehne 50. In dem dargestellten Ausführungsbeispiel findet eine positive Korrelation zwischen dem Parameter für die Aktivierung (Zeitdauer eines statischen Zustandes) und dem Parameter für die Deaktivierung (Range-Of-Motion) statt.

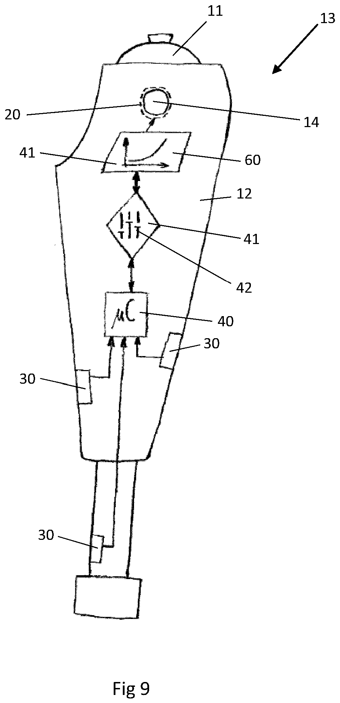

In der Figur 9 ist in einer schematischen Darstellung ein orthopädietechnisches Gelenk 13 in Gestalt eines Prothesenkniegelenkes gezeigt. Das Prothesenkniegelenk 13 weist ein Oberteil 11 und ein Unterteil 12 auf, die um eine Schwenkachse 14 drehbar aneinander gelagert sind. Das Unterteil 12 weist an seinem distalen Ende Anschlussmittel zur Befestigung eines Prothesenfußes auf. Ebenfalls sind mehrere Sensoren 30 an dem Unterteil 12 angeordnet, um Informationen und Zustandsdaten zu erfassen. Beispielsweise können Raumlageinformationen, Axialkräfte, Momente, Winkel und/oder Beschleunigungen über die Sensoren 30 gemessen werden. Die Sensorwerte werden einer Steuerungseinrichtung 40 übermittelt, in der die für die Verarbeitung der Sensorsignale notwendigen Einrichtungen vorhanden sind. Dies sind insbesondere Prozessoren, Speichereinrichtungen, Software, Energiespeicher sowie gegebenenfalls Sender und Empfänger zur Entgegennahme und Weiterleitung von Signalen. Die aufbereiteten Sensorsignale dienen zur Steuerung der Widerstandseinrichtung 20, die in dem dargestellten Ausführungsbeispiel als eine Bremseinrichtung ausgebildet ist, die eine Verschwenkung des Oberteils 11 relativ zu dem Unterteil 12 beeinflusst. An dem Unterteil 12 ist darüber hinaus ein Bedienelement 42 angeordnet, das ein Bedienfeld aufweisen kann oder an dem Schieberegler oder Drehknöpfe zur Einstellung von Parametern oder Schwellwerte der Parameter angeordnet oder ausgebildet sind. In dem dargestellten Ausführungsbeispiel sind drei Bedienelemente in einer Anzeigeeinrichtung 41 in Gestalt eines Displays abgebildet. Ein weiteres Display 41 kann im Bereich der Schwenkachse 14 angeordnet sein, in dem die Veränderung der Schwellwerte angezeigt wird. Dem Display 41 ist eine Verstelleinrichtung 60 zugeordnet, über die der Widerstand der Widerstandseinrichtung 20 verstellt werden kann. Das Display 41 kann auch an einer anderen Stelle des Prothesenkniegelenkes oder der orthopädietechnischen Einrichtung angeordnet sein und drahtlos mit der Verstelleinrichtung 60 kommunizieren, sodass eine Einstellung bequem erreichbar für den Nutzer an einem beliebigen Ort stattfinden kann, beispielsweise auch über ein Mobiltelefon, ein Tablet oder eine andere grafische Benutzeroberfläche.

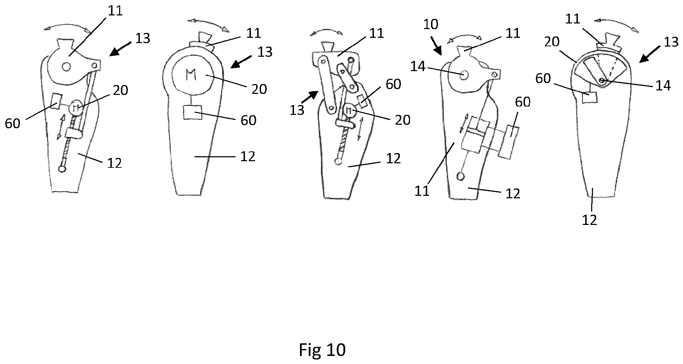

In der Figur 10 sind fünf Varianten von Verstelleinrichtungen und Widerstandseinrichtungen anhand von Prothesenkniegelenken dargestellt, entsprechende Komponenten können auch an Orthesen oder Exoskeletten angeordnet sein. Alle Prothesenkniegelenke 13 weisen ein Oberteil 11 und ein Unterteil 12 auf. In der linken Darstellung ist die Widerstandseinrichtung 20 als ein Spindelgetriebe ausgebildet, das einen Motor aufweist, der über die Verstelleinrichtung 60 aktivierbar und deaktivierbar ist. Dadurch wird die Verschwenkung des Oberteils 11 relativ zu dem Unterteil 12 verändert und gegebenenfalls in zumindest einer Richtung gesperrt.

In der zweiten Ausführungsform von links ist die Widerstandeinrichtung 20 als ein Motor ausgebildet, der unmittelbar an dem Oberteil 11 angeordnet ist und sich gegenüber dem Unterteil 12 abstützt. Der Motor stellt die Widerstandseinrichtung 20 dar und kann über eine entsprechende Steuerung von der Verstelleinrichtung 60 unterschiedlich bestromt werden, sodass unterschiedliche Widerstände gegen eine Flexion und/oder Extension bzw. eine Sperrung in einer der Richtungen bereitgestellt werden können.

In der mittleren Darstellung ist die Gelenkeinrichtung 13 als ein Mehrlenkersystem mit vier Lenkern und einer instationären Gelenksachse ausgebildet. Die Widerstandseinrichtung 20 ist als Spindelgetriebe mit einem Motor ausgebildet, der über die Verstelleinrichtung 60 aktiviert oder deaktiviert werden kann, um eine Verschwenkung des Oberteils 11 relativ zu dem Unterteil 12 zu beeinflussen oder eine Sperrung zu bewirken.

In der vierten Ausführungsform von links ist die Widerstandseinrichtung 20 als ein Hydraulikdämpfer oder Pneumatikdämpfer ausgebildet, der einen Zylinder aufweist, in dem ein Kolben mit einer Kolbenstange längsverschieblich geführt ist. Der Kolben unterteilt den Zylinder in eine Extensionskammer und eine Flexionskammer, die über einen Verbindungskanal miteinander gekoppelt sind. Innerhalb des Verbindungskanals befindet sich zumindest ein Stellventil, das hinsichtlich des Durchlassquerschnittes veränderlich ausgebildet ist. Über die Verstelleinrichtung 60 kann der Strömungsquerschnitt verändert oder eine strömungstechnische Verbindung von der Extensionskammer zur Flexionskammer gesperrt werden, um so eine Verschwenkbarkeit um die Schwenkachse 14 zu beeinflussen bzw. zu sperren.

In der rechten Darstellung der Figur 10 ist die Widerstandseinrichtung 20 als ein Schwenkkolbensystem ausgebildet, in dem sich der Schwenkkolben in einer Kammer befindet und korrespondierend mit der Verschwenkung des Oberteils 11 relativ zu dem Unterteil 12 verlagert werden kann. Der Schwenkkolben bildet ebenfalls eine Extensionskammer und eine Flexionskammer aus. Innerhalb des Schwenkkolbensystems kann sich eine magnetorheologische Flüssigkeit befinden, deren Viskosität über die Verstelleinrichtung 60 in Gestalt eines aktivierbaren und deaktivierbaren Magnetfeldes verändern lässt. Dadurch wird die Durchflussmenge von der Extensionskammer zu der Flexionskammer und umgekehrt in Abhängigkeit von dem angelegten Magnetfeld und der dadurch eingestellten Viskosität des Mediums beeinflusst.

## Patentansprüche

1. Orthopädietechnisches Gelenk mit einem Oberteil (11, 11') und einem schwenkbar daran angeordneten Unterteil (12, 12') und einer zwischen dem Oberteil (11, 11') und dem Unterteil (12, 12') angeordneten Widerstandseinrichtung (20), die einen Widerstand gegen eine Verschwenkbewegung des Oberteils (11, 11') zu dem Unterteil (12, 12') um eine Schwenkachse (14, 14') bereitstellt und eine Verstelleinrichtung (60) aufweist, über die der Widerstand einstellbar ist, wobei die Verstelleinrichtung (60) mit einer Steuerungseinrichtung (40) gekoppelt ist, die mit zumindest einem Sensor (30) gekoppelt ist, so dass über die Steuerungseinrichtung (40) der Widerstand auf Basis von Sensordaten, die von dem zumindest einen Sensor (30) an die Steuerungseinrichtung (40) übermittelt werden, verstellbar ist, in der Steuerungseinrichtung (40) ist eine Funktion hinterlegt, in der das Gelenk (13, 13') in Abhängigkeit von den Sensordaten gegen eine Verschwenkung in zumindest einer Richtung gesperrt ist, die Funktion ist auf Grundlage der Sensordaten für eine Sperrung aktivierbar und eine Entsperrung deaktivierbar, **dadurch gekennzeichnet, dass** zumindest ein Parameter für die Aktivierung und zumindest ein Parameter, der von dem Parameter für die Aktivierung verschieden ist, für die Deaktivierung der Funktion miteinander korreliert sind.

2. Orthopädietechnische Gelenkeinrichtung nach Anspruch 1, **dadurch gekennzeichnet, dass** als Parameter für die Aktivierung der Funktion die Zeitdauer eines statischen Zustandes des Gelenkes (13, 13') hinterlegt ist.

3. Orthopädietechnische Gelenkeinrichtung nach Anspruch 1 oder 2, **dadurch gekennzeichnet, dass** als Parameter für die Aktivierung oder Deaktivierung der Funktion eine Bewegung des Unterteils (12, 12'), des Oberteils (11, 11')oder eine Belastung des Gelenkes (13, 13')oder Änderungsraten der Bewegung oder Belastung hinterlegt ist.

4. Orthopädietechnisches Gelenk nach einem der voranstehenden Ansprüche, **dadurch gekennzeichnet, dass** als Parameter für die Deaktivierung der Funktion eine Raumlage des Oberteils (11, 11'), des Unterteils (12, 12') und/oder einer Verbindungslinie (50) zwischen zwei Punkten (51, 52) proximal und distal der Schwenkachse (14, 14') hinterlegt ist.

5. Orthopädietechnisches Gelenk nach einem der voranstehenden Ansprüche, **dadurch gekennzeichnet, dass** Schwellwerte für die Parameter für die Aktivierung und/oder Deaktivierung hinterlegt sind und die Schwellwerte veränderlich sind.

6. Orthopädietechnisches Gelenk nach Anspruch 5, **dadurch gekennzeichnet, dass** die Schwellwerte für die Aktivierung und Deaktivierung positiv korreliert sind.

7. Orthopädietechnisches Gelenk nach einem der voranstehenden Ansprüche, **dadurch gekennzeichnet, dass** die Steuerungseinrichtung (40) mit einer Anzeigeeinrichtung (41) und einem Bedienelement (42) zur Veränderung der Parameter oder der Schwellwerte der Parameter verbunden ist.

8. Orthopädietechnisches Gelenk nach einem der voranstehenden Ansprüche, **dadurch gekennzeichnet, dass** ein Gyroskop, ein Winkelsensor, ein Kraftsensor und/oder ein Raumlagesensor mit der Steuerungseinrichtung (40) gekoppelt ist.

9. Verfahren zur Steuerung eines orthopädietechnischen Gelenkes (13, 13') mit einem Oberteil (11, 11') und einem schwenkbar daran angeordneten Unterteil (12, 12') und einer zwischen dem Oberteil (11, 11') und Unterteil (12, 12') angeordneten Widerstandseinrichtung (20), die einen Widerstand gegen eine Verschwenkbewegung des Oberteils (11, 11') zu dem Unterteil (12, 12') um eine Schwenkachse (14, 14') bereitstellt und eine Verstelleinrichtung (60) aufweist, über die der Widerstand auf Basis von Sensordaten, die von zumindest einem Sensor (30) an eine mit der Verstelleinrichtung (60) verbundenen Steuerungseinrichtung (40) übermittelt werden, dergestalt verstellt wird, dass bei Aktivierung einer Funktion das Gelenk (20) gegen eine Verschwenkung gesperrt und bei Deaktivierung der Funktion entsperrt wird, **dadurch gekennzeichnet, dass** zumindest ein Schwellwert für einen Parameter für die Aktivierung und zumindest ein Schwellwert für einen Parameter, der von dem Parameter für die Aktivierung verschieden ist, für die Deaktivierung der Funktion miteinander korreliert werden.

10. Verfahren nach Anspruch 9, **dadurch gekennzeichnet, dass** die Schwellwerte positiv miteinander korreliert werden.

11. Verfahren nach Anspruch 9 oder 10, **dadurch gekennzeichnet, dass** die Funktion auf Grundlage der Zeitdauer eines statischen Zustandes der Gelenkeinrichtung aktiviert wird.

12. Verfahren nach einem der Ansprüche 9 bis 11, **dadurch gekennzeichnet, dass** die Funktion auf der Grundlage einer Bewegung des Unterteils (12, 12'), des Oberteils (11, 11') und/oder eine Belastung des Gelenkes (13, 13') aktiviert oder deaktiviert wird.

13. Verfahren nach einem der Ansprüche 9 bis 11, **dadurch gekennzeichnet, dass** die Funktion auf der Grundlage einer Raumlage des Oberteils (11, 11'), des Unterteils (12, 12') und/oder einer Verbindungslinie (50) zwischen zwei Punkten (51, 52) proximal und distal der Schwenkachse (14, 14') deaktiviert wird.

14. Verfahren nach einem der Ansprüche 9 bis 13, **dadurch gekennzeichnet, dass** die Schwellwerte für die Parameter für die Aktivierung und Deaktivierung veränderlich sind.

## Claims

1. An orthopedic joint comprising an upper part (11, 11') and a lower part (12, 12') pivotably arranged on the latter, and a resistance device (20) which is arranged between the upper part (11, 11') and the lower part (12, 12') and provides resistance against a pivoting movement of the upper part (11, 11') relative to the lower part (12, 12') about a pivot axis (14, 14'), and which has an adjustment device (60) by means of which the resistance is adjustable, wherein the adjustment device (60) is coupled to a control device (40), which is coupled to at least one sensor (30), such that the resistance is adjustable by means of the control device (40) on the basis of sensor data that are transmitted from the at least one sensor (30) to the control device (40), wherein the control device (40) has stored in it a function in which the joint (13, 13') is locked against pivoting in at least one direction in accordance with the sensor data, and the function can be activated for locking and deactivated for unlocking on the basis of the sensor data, **characterized in that** at least one parameter for activating the function and at least one parameter for deactivating the function , which is different from the parameter for activation, are correlated with one another.

2. The orthopedic joint device as claimed in claim 1, **characterized in that** the duration of a static state of the joint (13, 13') is stored as a parameter for activating the function.

3. The orthopedic joint device as claimed in claim 1 or 2, **characterized in that** a movement of the lower part (12, 12'), a movement of the upper part (11, 11') or a loading of the joint (13, 13') or rates of change of the movement or loading are stored as parameters for activating or deactivating the function.

4. The orthopedic joint as claimed in one of the preceding claims, **characterized in that** a spatial position of the upper part (11, 11'), a spatial position of the lower part (12, 12') and/or a connecting line (50) between two points (51, 52) located proximally and distally of the pivot axis (14, 14') are stored as parameters for deactivating the function.

5. The orthopedic joint as claimed in one of the preceding claims, **characterized in that** threshold values for the parameters for activation and/or deactivation are stored, and the threshold values are variable.

6. The orthopedic joint as claimed in claim 5, **characterized in that** the threshold values for activation and deactivation are positively correlated.

7. The orthopedic joint as claimed in one of the preceding claims, **characterized in that** the control device (40) is connected to a display device (41) and to an operating element (42) for changing the parameters or the threshold values of the parameters.

8. The orthopedic joint as claimed in one of the preceding claims, **characterized in that** a gyroscope, an angle sensor, a force sensor and/or a spatial position sensor is coupled to the control device (40).

9. A method for controlling an orthopedic joint (13, 13') comprising an upper part (11, 11') and a lower part (12, 12') pivotably arranged on the latter, and a resistance device (20) which is arranged between the upper part (11, 11') and the lower part (12, 12') and provides resistance against a pivoting movement of the upper part (11, 11') relative to the lower part (12, 12') about a pivot axis (14, 14'), and which has an adjustment device (60) by means of which the resistance is adjusted on the basis of sensor data transmitted from at least one sensor (30) to a control device (40) connected to the adjustment device (60), in such a way that the joint (20) is locked against pivoting upon activation of a function and unlocked upon deactivation of the function, **characterized in that** at least one threshold value for a parameter for activating the function and at least one threshold value for a parameter for deactivating the function , which is different from the parameter for activation, are correlated with one another.

10. The method as claimed in claim 9, **characterized in that** the threshold values are positively correlated with one another.

11. The method as claimed in claim 9 or 10, **characterized in that** the function is activated on the basis of the duration of a static state of the joint device.

12. The method as claimed in one of claims 9 through 11, **characterized in that** the function is activated or deactivated on the basis of a movement of the lower part (12, 12'), a movement of the upper part (11, 11') and/or a loading of the joint (13, 13').

13. The method as claimed in one of claims 9 through 11, **characterized in that** the function is deactivated on the basis of a spatial position of the upper part (11, 11'), a spatial position of the lower part (12, 12') and/or a connecting line (50) between two points (51, 52) located proximally and distally of the pivot axis (14, 14').

14. The method as claimed in one of claims 9 through 13, **characterized in that** the threshold values for the parameters for activation and deactivation are variable.

## Revendications

1. Articulation orthopédique comprenant une partie supérieure (11, 11') et une partie inférieure (12, 12') disposée de manière pivotante sur celle-ci et un dispositif de résistance (20) disposé entre la partie supérieure (11, 11') et la partie inférieure (12, 12'), lequel assure une résistance au mouvement de pivotement de la partie supérieure (11, 11') par rapport à la partie inférieure (12, 12') autour d'un axe de pivotement (14, 14') et présente un dispositif de réglage (60) permettant de régler la résistance, le dispositif de réglage (60) étant couplé à un dispositif de commande (40) qui est couplé à au moins un capteur (30), de sorte que la résistance peut être réglée par l'intermédiaire du dispositif de commande (40) sur la base de données de capteur qui sont transmises par ledit au moins un capteur (30) au dispositif de commande (40), une fonction étant enregistrée dans le dispositif de commande (40), dans laquelle l'articulation (13, 13') est bloquée à l'encontre d'un pivotement dans au moins une direction en réponse aux données du capteur, la fonction pouvant être activée pour un blocage et être désactivée pour un déblocage sur la base des données du capteur,
**caractérisée en ce qu'**au moins un paramètre d'activation de la fonction et au moins un paramètre de désactivation, différent du paramètre d'activation, sont corrélés entre eux.

2. Dispositif d'articulation orthopédique selon la revendication 1,
**caractérisé en ce que** la durée d'un état statique de l'articulation (13, 13') est enregistrée comme paramètre d'activation de la fonction.

3. Dispositif d'articulation orthopédique selon la revendication 1 ou 2,
**caractérisé en ce qu'**un mouvement de la partie inférieure (12, 12'), de la partie supérieure (11, 11') et/ou une charge sur l'articulation (13, 13') et/ou des taux de variation du mouvement ou de la charge sont enregistrés comme paramètres d'activation ou de désactivation de la fonction.

4. Articulation orthopédique selon l'une des revendications précédentes,
**caractérisée en ce qu'**une position spatiale de la partie supérieure (11, 11'), de la partie inférieure (12, 12') et/ou d'une ligne de liaison (50) entre deux points (51, 52) du côté proximal et distal de l'axe de pivotement (14, 14') est enregistrée comme paramètre de désactivation de la fonction.

5. Articulation orthopédique selon l'une des revendications précédentes,
**caractérisée en ce que** des valeurs seuils sont enregistrées pour les paramètres d'activation et/ou de désactivation, et les valeurs seuils sont variables.

6. Articulation orthopédique selon la revendication 5,
**caractérisée en ce que** les valeurs seuils pour l'activation et la désactivation sont corrélées positivement.

7. Articulation orthopédique selon l'une des revendications précédentes,
**caractérisée en ce que** le dispositif de commande (40) est relié à un dispositif d'affichage (41) et à un élément de manipulation (42) pour modifier les paramètres ou les valeurs seuils des paramètres.

8. Articulation orthopédique selon l'une des revendications précédentes,
**caractérisée en ce qu'**un gyroscope, un capteur d'angle, un capteur de force et/ou un capteur de position spatiale est couplé au dispositif de commande (40).

9. Procédé de commande d'une articulation orthopédique (13, 13') comprenant une partie supérieure (11, 11') et une partie inférieure (12, 12') disposée de manière pivotante sur celle-ci et un dispositif de résistance (20) disposé entre la partie supérieure (11, 11') et la partie inférieure (12, 12'), lequel assure une résistance au mouvement de pivotement de la partie supérieure (11, 11') par rapport à la partie inférieure (12, 12') autour d'un axe de pivotement (14, 14') et présente un dispositif de réglage (60) permettant de régler la résistance sur la base de données de capteur qui sont transmises par au moins un capteur (30) à un dispositif de commande (40) relié au dispositif de réglage (60), de telle sorte que, lors de l'activation d'une fonction, l'articulation (20) est bloquée à l'encontre d'un pivotement et, lors de la désactivation de la fonction, elle est débloquée,
**caractérisé en ce qu'**au moins une valeur seuil pour un paramètre d'activation de la fonction et au moins une valeur seuil pour un paramètre de désactivation, différent du paramètre d'activation, sont corrélées entre elles.

10. Procédé selon la revendication 9,
**caractérisé en ce que** les valeurs seuils sont corrélées positivement entre elles.

11. Procédé selon la revendication 9 ou 10,
**caractérisé en ce que** la fonction est activée sur la base de la durée d'un état statique du dispositif d'articulation.

12. Procédé selon l'une des revendications 9 à 11,
**caractérisé en ce que** la fonction est activée ou désactivée sur la base d'un mouvement de la partie inférieure (12, 12'), de la partie supérieure (11, 11') et/ou d'une charge sur l'articulation (13, 13').

13. Procédé selon l'une des revendications 9 à 11,
**caractérisé en ce que** la fonction est désactivée sur la base d'une position spatiale de la partie supérieure (11, 11'), de la partie inférieure (12, 12') et/ou d'une ligne de liaison (50) entre deux points (51, 52) du côté proximal et distal de l'axe de pivotement (14, 14').

14. Procédé selon l'une des revendications 9 à 13,
**caractérisé en ce que** les valeurs seuils des paramètres d'activation et de désactivation sont variables.
